# EUROPEAN PATENT SPECIFICATION

(11) **EP 0 602 049 B1**
(45) Date of publication and mention of the grant of the patent: **08.09.1999**
(21) Application number: 92914059.8
(22) Date of filing: 06.07.1992
(51) Int. Cl.: C12Q 1/68, G01N 33/535

(54) **NUCLEIC ACIDS ASSAY**
NUKLEINSÄURENACHWEIS
DOSAGE D'ACIDES NUCLEIQUES

(30) Priority: 05.07.1991 GB 9114525
(43) Date of publication of application: 22.06.1994
(73) Proprietor: CYTOCELL LIMITED, Adderbury, Banbury, Oxon OX17 3SN (GB)
(72) Inventor: CARDY, Donald Leonard Nicholas, "Trinlan", Northamptonshire NN11 6UF (GB); DELNATTE, Sabine Yolande Joseph, Lewknor, Oxfordshire OX9 5TS (GB)
(74) Representative: Matthews, Heather Clare
(86) International application number: GB9201229
(87) International publication number: WO9301313

(56) References cited:
- EP-A- 0 144 913
- EP-A- 0 144 914
- EP-A- 0 149 339
- EP-A- 0 232 967
- EP-A- 0 309 230
- EP-A- 0 330 185
- EP-A- 0 343 955
- WO-A-86/02666

## Description

The present invention relates to methods for homogeneous nucleic acid probe-based tests for genetic materials whereby the formation of a nucleic acids hybrid modulates a signal-producing moiety or intermediate resulting in production of or a change in the signal. In particular, the present invention relates to methods whereby the formation of a nucleic acids hybrid modulates the activity of an enzyme or an enzyme intermediate. The present invention also relates to substances required for such homogeneous nucleic acids-based tests.

In the current state-of-the-art, laboratory nucleic acid probe-based assays for detection of genetic material, for example from living organisms, usually require the purification of genetic material from these organisms followed by its immobilisation on a solid phase, typically a nitrocellulose membrane. The membrane is then usually incubated with a DNA probe labelled with either a radioactive moiety (for example, phosphorous-32), an enzyme (for example, alkaline phosphatase) or any other signal producing moiety or intermediate. The membrane is then washed to remove non-hybridised DNA probe before undertaking the appropriate steps for analysis of signal associated with the membrane (for example autoradiography for phosphorous-32, addition of an enzyme substrate for alkaline phosphatase). For detailed protocols for such assays, reference may be made to Molecular Cloning, A Laboratory Manual, (eds. Sambrook, Fritsch and Maniatis, Cold Spring Harbor 1989). Such laboratory probe-based assays can be termed "heterogeneous DNA probe assays" whereby, following hybridisation, the hybridised DNA probe must be separated from the unhybridised DNA probe in the solution phase in order to determine the presence of specific genetic material as measured by signal associated with the membrane

Such heterogeneous nucleic acid probe-based assays require a number of different manipulation steps including extensive washing of the solid phase in order to remove excess probe. The large number of steps makes automation or routine use difficult such that heterogeneous assays have not been widely introduced for analyses (for example, in routine clinical testing).

Nucleic acid probe-based assays where no separation of hybridised and unhybridised DNA probe is required for signal generation can be termed "homogeneous DNA probe assays". The main principle of such assays is that hybridised probe gives rise to a different signal from unhybridised probe such that separation of hybridised and unhybridised probe is unnecessary. Thus, simply by measuring the development of the modified signal resultant from hybridisation can the presence of genetic material be determined. Such homogeneous assays therefore avoid any need for separation or washing steps such that suitable preparations of genetic material can be simply mixed with one or more reagents in succession to produce the final modified signal. Such separation steps are a major cause of variability in assay results, are time and labour consuming, require additional equipment and make automation more difficult and expensive. Prospectively, homogeneous nucleic acid probe assays will be simple enough for routine clinical testing and other routine applications and will readily lend themselves to automation using, for example, existing high-throughput clinical analysers in cases where analysers are capable of measuring the final modified signal from the homogeneous assay. There is currently an unsatisfied need for simple homogeneous nucleic acids probe-based assays.

Several methods for homogeneous DNA probe assays have been described in the patent or scientific literature. Invariably, these fall within two categories, either assays where the signal derives from the interaction of a molecule on one probe with that on another probe, or assays where the process of hybridisation affects or creates one or more signal producing moieties attached to the DNA probe. Thus EP144914 assigned to Miles describes the interaction of a molecule on one DNA probe with a molecule on an adjacently hybridising DNA probe whereby the molecules interact through enzyme channelling or fluorescence energy transfer to produce a signal. EP232967 assigned to Amoco provides a competitive assay variation of EP144914 whereby the two DNA probes normally hybridise together to produce a signal but where a test nucleic acid competes for hybridisation with one probe to produce a different signal (fluorescence in the example provided). EP229943 assigned to Molecular Biosystems appears to be a more specific variation of EP144914 comprising fluorescence energy transfer between adjacently hybridised probes.

Patents whereby hybridisation affects or creates signalling are exemplified by EP144913 assigned to Miles whereby species recognised by an antibody are created by hybridisation of a nucleic acid probe to its target. EP231495 assigned to Enzo appears to broaden this concept to produce a "property change" upon hybridisation which is the signal. One example given is the alteration in fluorescence of a dye conjugated to a DNA probe which intercalates into hybrids formed to give a modified fluorescence signal. A novel hybridisation associated "change" is described by Vary (Nucl. Acids Res. 15 (1987) p6883) whereby the hybridisation of target DNA to a DNA probe already hybridised to a RNA strand is possible through the displacement of the RNA strand to produce a single-stranded RNA species. Such a single-stranded RNA species is then susceptible to hydrolysis by polynucleotide phosphorylase with the release of ADP. Subsequently, ADP can be converted to ATP for estimation by luciferase-catalysed chemiluminescence.

Whilst each of the above described homogeneous nucleic acid probe methodologies are conceptually elegant, none has yet proved suitable for commercialisation. The major difficulties include non-specific hybridisation associated signalling changes and the general limitations, where applicable, of fluorescence measurements particulary at wavelengths which are finely restricted to exclude other fluorescences and where background and variability are problems. The only homogeneous DNA probe assay to achieve a level of commercial success is disclosed in EP309230 assigned to ML Technology Ventures. This has been successfully adapted by Genprobe Corporation whereby fluorescent acridinium ester moieties attached to a DNA probe are selectively degraded in unhybridised probe but protected in hybrids formed by the acridinium-labelled probe. Whilst this fluorescence assay has been successfully adapted for detection of the abundant ribosomal RNA from microorganisms such as C. trachomatis and N. gonorrhoeae, the sensitivity of the assay will limit many other DNA probe applications especially for analysis of single copy mammalian genes. From the precedent of antibody-based assays (i.e. immunoassays), the key to higher sensitivities is an assay involving an enzyme-generated signal where the enzyme product can either be allowed to accumulate to give extra sensitivity or can itself be used as a substrate or cofactor for another enzyme activity.

The concept of homogeneous assays is well known in the field of immunoassays (i.e. assays involving antibodies) whereby the binding of one or more antibodies to a specific analyte results in development or modification of a signal producing moiety. Nevertheless, few homogeneous immunoassay systems have proved commercially viable and none readily lend themselves to nucleic acid probe-based assays. The enzyme-labelled systems commercialised by the Syva company (Rubenstein et al, US Patent No 3817837, Rubenstein and Ullman, US Patent No 3875011) involve the binding of a low molecular weight analyte to an enzyme whereby an antibody which binds to the analyte modulates the signal produced by the enzyme. These and related Syva patents (for example US 3817837, US3852157, US3875011, US3905871, US3966556, US4039385, US4040907, US4043872, US4046636, US4065354, US4067774, US4171244 and US 4191613) do not teach how to apply such processes to nucleic acid hybridisation assays nor is such application obvious. The substrate-labelled systems commercialised by the Ames company (Burd et al, Clin. Chem. 23 (1977) p1402 and Anal. Biochem. 77 (1977) p56) involve the production of a non-fluorescent analyte conjugate whose hydrolysis to yield a fluorescent moiety can be blocked by an analyte specific antibody. This system is also limited to low molecular weight analytes and does not obviously apply to nucleic acids hybridisations. The fluorescence polarisation system commercialised by the Abbott company (Dandliker et al, Imnunochemistry 10 (1973) p219) is similar in concept to the Ames system except the the analyte is conjugated to a fluorescent molecule whose fluorescence is modified directly as a change in polarisation through binding of an antibody specific for the analyte. The enzyme inhibitor system of Kallestad (Bacquet and Twumasi, Anal. Biochem. 136 (1984) p487) involves the inhibition of biotin-dependant enzymes by analyte-conjugated avidin and reversal of this inhibition by an antibody specific for the analyte. Both the Abbott and Kallestad systems suffer from low sensitivity (analyte concentrations usually >10⁻¹⁰M), by application limited to low molecular weight analytes (usually <1000 daltons), and by no obvious application to nucleic acid hybridisation assays.

One successful homogeneous immunoassay system which has improved sensitivity and has application to large molecular weight analytes has been commercialised by the Microgenics company (Henderson, EP 199801 and PCT 86/02666). This is based on a feature of relatively few enzymes whose activity can be reconstituted by the recombination of inactive fragments of enzyme. In this assay system, the analyte is attached to a peptide fragment from beta-galactosidase which can activate enzyme activity when mixed with the remaining beta-galactosidase protein. Analyte-specific antibody can interfere with this recombination to preclude enzyme activity whilst added analyte will compete for antibody binding to the peptide-analyte fragment to allow enzyme activation in an analyte concentration-dependant fashion. This assay system is not restricted to low molecular weight analytes and has a sensitivity >10⁻¹⁰M). However from EP199801 and related patents, it is not obvious how to apply this assay system for nucleic acid hybridisations. Indeed, the claims refer to an analyte binding protein which directly competes with the beta-galactosidase "enzyme-acceptor" (i.e. the inactive partial beta-galactosidase protein) with the peptide-analyte conjugate. Neither the claims nor the patent description indicate how the process of nucleic acid hybridisation could interfere with activation of enzyme activity by the beta-galactosidase peptide.

Despite the precedents from the immunoassay field, no enzyme-based homogeneous nucleic acid hybridisation-based assays are known in the literature and thus there is a great need for such assays. Thus, the present invention comprises methods for homogeneous nucleic acid probe-based hybridisation assays based on modulation of enzyme activity. The key finding in relation to the invention is that nucleic acid probes can either be attached to a moiety which activates an enzyme or directly to the enzyme itself without respectively abolishing the enzyme activation or the enzyme activity, and which in principle can be attached to a moiety which inhibits an enzyme without abolishing inhibition of enzyme activity. The invention then provides the means for modulating enzyme activity through the hybridisation of derivatised nucleic acid probes whereby moieties attached to the probe either directly or indirectly interfere respectively with enzyme activity, activation or inhibition.

One embodiment of the current invention provides a means to modulate the reconstitution of an active enzyme by nucleic acid hybridisation. A key finding in the development of this "homogeneous DNA probe-based enzyme activation assay" is that nucleic acids can, by certain methods, be attached to an inactive peptide component of an enzyme without precluding the ability of that peptide to activate enzyme activity. Another key finding is that hybridisation of unmodified nucleic acids alone to this nucleic acid-peptide conjugate is not sufficient to totally preclude enzyme activation (although larger nucleic acid molecules can reduce activation). Thus, unlike in the immunoassay system described in EP199801, the "analyte" (nucleic acid)-peptide conjugate in a nucleic acid probe-based assay cannot be directly inhibited from reconstituting enzyme activity by hybridisation with a complementary sample-derived nucleic acid analyte molecule. In the immunoassay system of EP199801, the analyte-peptide conjugate is inhibited from reconstituting enzyme activity by an "analyte-binding protein", invariably an antibody, which binds to the analyte both in the analyte-peptide conjugate or as free analyte. When free analyte is introduced as in a test sample, then a proportion of the antibody is diverted to binding to free analyte leaving a proportion of the analyte-peptide conjugate free to reconstitute enzyme activity. For this system to be applied directly to nucleic acids, an analyte-binding protein would be needed which recognises a nucleic acid-peptide conjugate together with the free nucleic acid. It is well understood that development of an antibody (or any other protein) with high specificity for a specific nucleic acid sequence is very difficult and thus alternative non-obvious ways would be needed to apply the system of EP199801 to nucleic acids. During the development of the present invention, it was considered possible that a synthetic nucleic acid molecule with a nucleotide sequence complementary to that of the nucleic acid-peptide conjugate might inhibit reconstitution of enzyme activity; it was also considered that, in turn, this inhibition might be reversed by the introduction of a free nucleic acid from a test sample with the same or a similar sequence as the synthetic nucleic acid molecule which would divert the synthetic nucleic acid molecule away from the nucleic acid-peptide conjugate. Thus, it was envisaged that the synthetic nucleic acid molecule would act in an analogous manner to the analyte-binding protein in EP199801 by inhibiting reconstitution of enzyme activity until sequestered by a test sample analyte. However, it was discovered that a synthetic complementary nucleic acid molecule, as with a complementary sample nucleic acid molecule, failed to efficiently inhibit the reconstitution of enzyme activity. Instead, it was discovered that the synthetic nucleic acid molecule with a nucleotide sequence complementary to that of the nucleic acid-peptide conjugate could be modified by certain methods to effectively inhibit reconstitution of enzyme activity. A preferred method is to locate a ligand molecule on the synthetic nucleic acid which is subsequently bound by an antibody which inhibits reconstitution, possibly by steric hindrance. Unlike in the immunoassay example, the antibody in the case of nucleic acids hybridisation does not bind directly to the analyte-peptide conjugate. An alternative method is to use an antibody or nucleic acid binding protein (or any other molecule) specific for single-stranded nucleic acid which will bind to the nucleic acid-peptide conjugate and inhibit reconstitution of enzyme activity. The hybridisation of a complementary test nucleic acid to the nucleic acid-peptide conjugate can then preclude antibody or protein binding thus allowing enzyme reconstitution. Unlike in the immunoassay example, the antibody (or binding moiety) in the case of nucleic acids hybridisation is not diverted from the analyte-peptide conjugate by binding to analyte from the sample but rather by the nucleic acid in the conjugate becoming double-stranded. An alternative method still is to employ a modified synthetic nucleic acid-peptide conjugate whereby an antibody binds to the specific modification and inhibits reconstitution of enzyme activity but whereby the hybridisation of a complementary test nucleic acid to the nucleic acid-peptide conjugate can then preclude antibody or protein binding thus allowing enzyme reconstitution. An alternative method still is to employ a modified synthetic nucleic acid molecule which, instead of hybridising directly to the nucleic acid-peptide conjugate, will hybridise adjacent to the nucleic acid-peptide conjugate on a contiguous strand of target nucleic acid whereby, as in the preferred method, an antibody can bind to a ligand on the modified nucleic acid molecule and inhibit enzyme reconstitution by the nucleic acid-peptide molecule.

In a first aspect the invention provides a homogeneous assay method for determining the presence of a nucleic acid sequence of interest in a sample, the method comprising the steps of:
(a) combining in solution the sample. a first probe comprising single stranded nucleic acid substantially complementary to at least part of the sequence of interest and associated with a substance capable of activating an enzyme. and a second probe having specific binding activity for the first probe when the first probe is not bound to the sequence of interest;
(b) adding an enzyme capable of being activated by the substance associated with the first probe. such that in the absence of the sequence of interest the second probe binds to the first probe, wherein such binding inhibits activation of the enzyme by the substance associated with the first probe; and
(c) monitoring said enzyme activity.

The invention also provides a homogeneous assay method for determining the presence of a nucleic acid sequence of interest in a sample, the method comprising the steps of:
(a) combining in solution the sample, a first probe comprising single stranded nucleic acid substantially complementary to at least part of the sequence of interest and associated with a substance capable of activating an enzyme, and a second probe comprising a ligand moiety and single stranded nucleic acid complementary to the first probe, such that the presence of the sequence of interest in the sample tends to inhibit binding of the second probe to the first probe;
(b) adding an enzyme which is activatable by the substance associated with the first probe, and a binding partner capable of binding to the ligand moiety, such that when the first and second probes are bound to each other, binding of the binding partner to the ligand moiety inhibits activation of the enzyme by the substance associated with the first probe; and
(c) monitoring said enzyme activity.

Further, the present invention provides a homogeneous assay method for determining the presence of a nucleic acid sequence of interest in a sample, the method comprising the steps of:
(a) combining in solution the sample. a first probe comprising single stranded nucleic acid substantially homologous with at least part of the sequence of interest and associated with a substance capable of activating an enzyme, and a second probe comprising a ligand moiety and single stranded nucleic acid complementary to both at least part of the sequence of interest and to the nucleic acid present in the first probe, such that the presence of the sequence of interest in the sample tends to inhibit binding of the second probe to the first probe;
(b) adding an enzyme which is activatable by the substance associated with the first probe, and a binding partner capable of binding to the ligand moiety, such that when the first and second probes are bound to each other, binding of the binding partner to the ligand moiety inhibits activation of the enzyme by the substance associated with the first probe; and
(c) monitoring said enzyme activity.

In addition the present invention provides a homogeneous assay method for determining the presence of a nucleic acid sequence of interest in a sample, the method comprising the steps of:
(a) combining in solution the sample, a first probe comprising single stranded nucleic acid substantially complementary to at least part of the sequence of interest and associated with a substance capable of activating an enzyme, and a second probe comprising a ligand moiety and single stranded nucleic acid complementary to at least part of the sequence of interest. such that the presence of the sequence of interest in the sample tends to cause binding of the first and second probes in close proximity to each other on the sequence of interest:
(b) adding an enzyme which is activatable by the substance associated with the first probe, and a binding partner capable of binding to the ligand moiety, such that when the first and second probes are bound in close proximity to each other, binding of the binding partner to the ligand moiety inhibits activation of the enzyme by the substance associated with the first probe; and
(c) monitoring said enzyme activity.

It will be apparent to those skilled in the art that the process of nucleic acids hybridisation with at least one modified nucleic acids probe could be used in many different ways to create a molecular species which can either directly or indirectly modulate an enzyme activity.

Specific embodiments of the invention will now be described by way of example with reference to the accompanying drawings in which:-
Figures 1 to 3, 12 and 13, and 14 and 15 illustrate homogeneous DNA probe-based enzyme activation assays whereby the signal is produced by activation of an enzyme;
Figures 4 to 6 illustrate a homogeneous DNA probe-based direct enzyme inhibition assay whereby the signal is produced by inhibition of an enzyme;
Figures 7 to 9 illustrate a homogeneous DNA probe-based indirect enzyme inhibition assay whereby the signal is produced by interference with the inhibition of an enzyme;
Figures 10 and 11 illustrate a homogeneous DNA probe-based assay whereby the signal results from the hybridisation-induced formation of a binding protein.

Figure 16 shows the amino acid sequence of the synthetic "cysteine/peptide" of beta-galactosidase as used for examples 1 and 2 below.

Figures 17 and 18 show the effect of sample human CFTR gene "PCR fragment" on the enzyme activation assays described in examples I.IV and I.VI respectively.

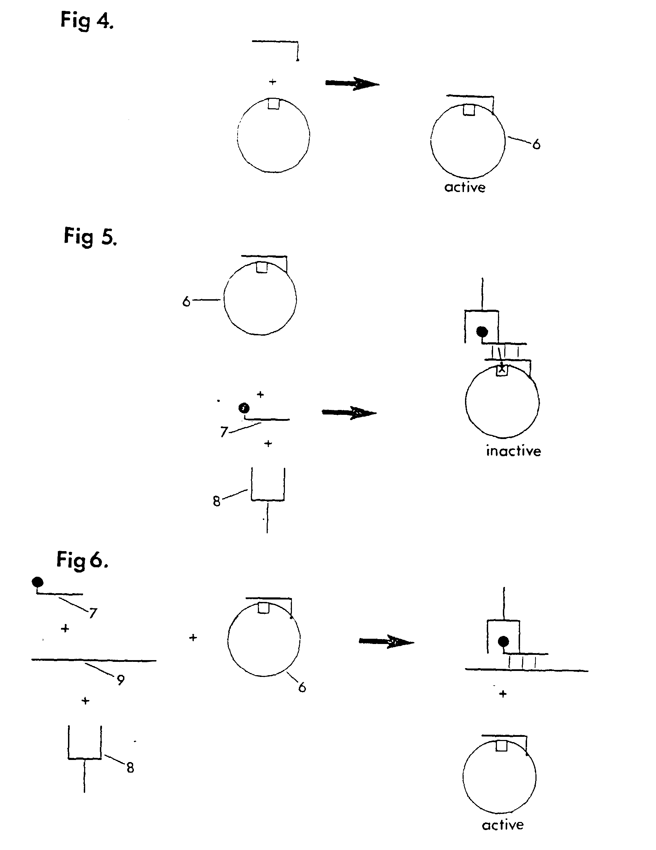

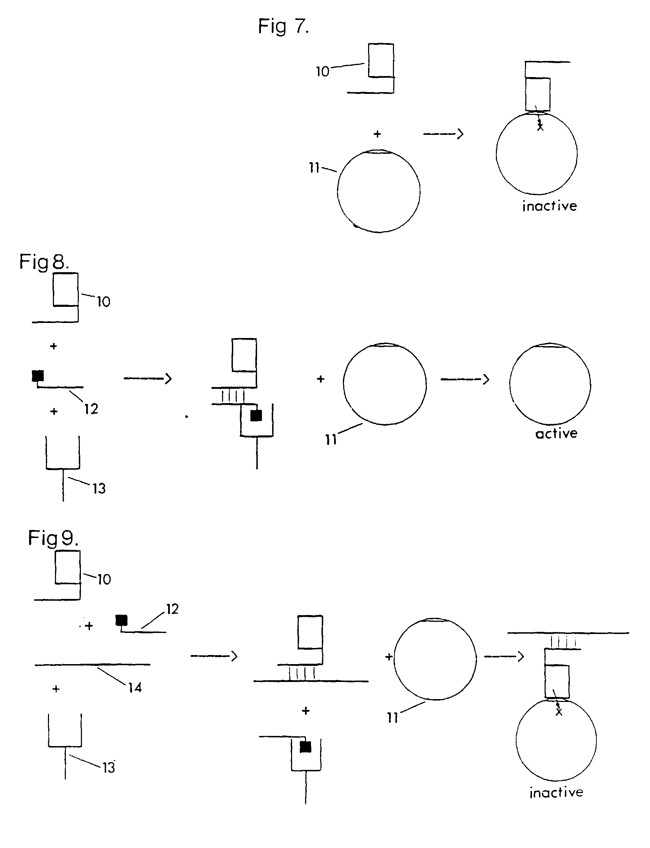

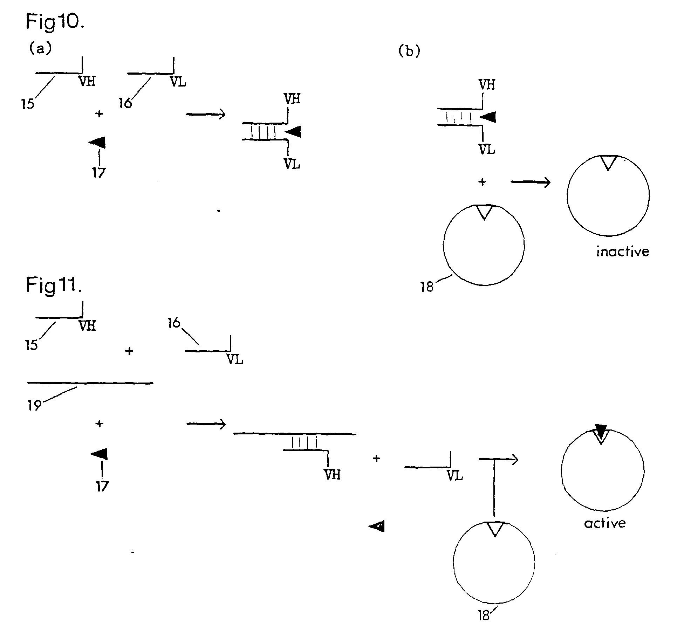

The embodiments shown in Figures 4-11 are considered to be outside the scope of the present invention.

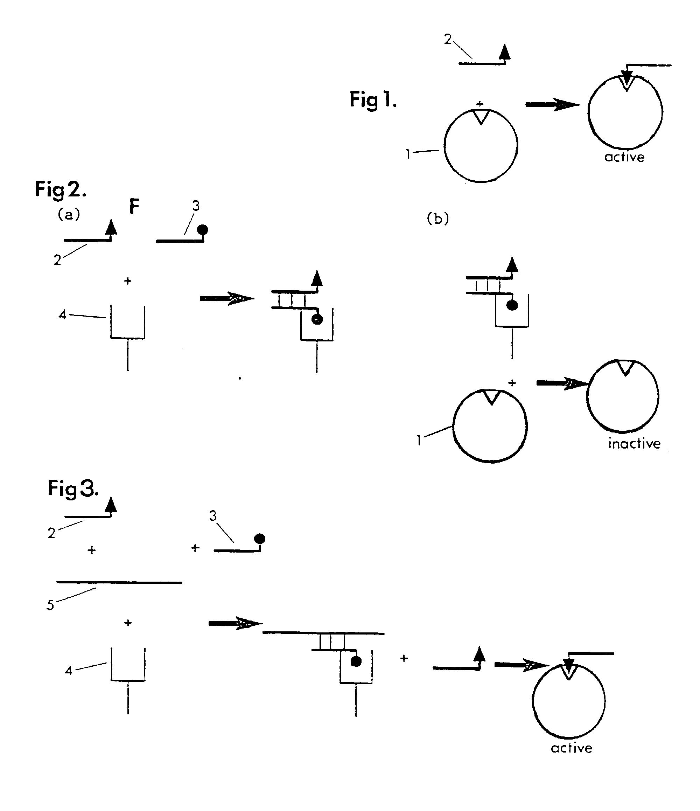

Referring to figure 1, one version of the enzyme activation assay comprises an inactive enzyme fragment 1 which can be activated by a peptide conjugated to a nucleic acid probe 2 to restore activity. Referring to figure 2, by hybridisation of a second nucleic acid probe 3 to which is attached a ligand which then becomes associated with a ligand-specific antibody 4, the activation of enzyme fragment 1 by the peptide conjugated nucleic acid probe 2 is inhibited. Referring to figure 3, by competition for hybridisation to ligand associated probe 3 by the test nucleic acids 5, the extent of hybridisation of probe 3 to probe 2 is thus reduced to leave a proportion of probe 2 unhybridised and thus able to activate enzyme fragment 1.

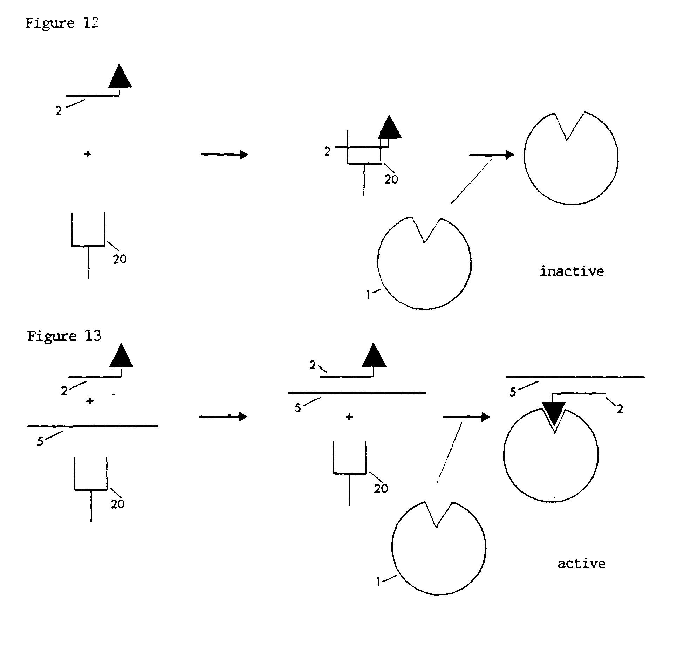

Referring to figure 12, another version of the enzyme activation assay comprises the same inactive enzyme fragment 1 as in figures 1 to 3 which can be activated by a peptide conjugated to a nucleic acid probe 2 to restore enzyme activity. In this case, a single-stranded nucleic acid specific antibody 20 (or other sequence-specific or non-specific molecule with appropriate binding specificity) is included which binds to the peptide conjugated nucleic acid 2 to inhibit enzyme activation. As in figure 13, the specific antibody 20 (or other molecule) are conversely unable to bind to peptide conjugated nucleic acid 2 when this becomes hybridised with target nucleic acid 5 and thus the peptide conjugated nucleic acid is free for activation of enzyme fragment 1.

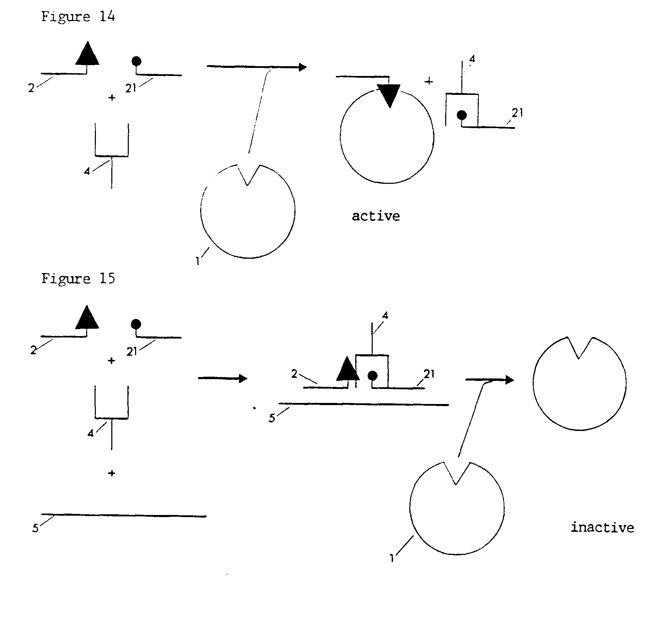

Referring to figure 14, another version of the enzyme activation assay comprises the same inactive enzyme fragment 1 as in figures 1 to 3 which can be activated by a peptide conjugated to a nucleic acid probe 2 to restore enzyme activity. In this case, a second nucleic acid probe 21 is included which is non-complementary to the peptide-nucleic acid 2 and to which is attached a ligand which then becomes associated with a ligand-specific antibody 4. However, as illustrated in figure 15, the activation of enzyme is only inhibited when the nucleic acid-peptide molecule 2 and the ligand-derivatised nucleic acid 21 hybridise adjacently to a target nucleic acid 5 whereby the binding of ligand-specific antibody 4 interferes with activation of the enzyme fragment 1 by the nucleic acid-peptide 2.

It will be understood to those skilled in the art that as alternatives to beta-galactosidase, other enzymes could be used where it is possible to split off a peptide with a loss of enzyme activity which is then automatically restored upon interaction of the peptide with the inactive enzyme fragment. For example, N-terminal peptide fragments of RNAase S can be removed and added back to restore full enzyme activity.

The homogeneous nucleic acid probe-based assays described herein circumvent many of the difficulties in achieving practical hybridisation assays particulary through avoidance of a solid phase. Assays may be undertaken by mixing together dilutions of target genetic material with nucleic acid probe, enzyme and antibody reagents and by recording enzyme activities at specific timepoints. Alternatively, samples and reagents may be loaded into an appropriate commercially available clinical analyser and the dilution, mixing and optical density measurements could then be undertaken by the machine.

The invention can be further understood from the following more detailed descriptions which are given by way of example and should not be regarded as limiting the scope of the invention.

### Example 1 - HOMOGENEOUS ENZYME ACTIVATION ASSAYS FOR CFTR GENES

In this example, the enzyme activation assays as referred to in figures 1 to 3 and 12 to 15 were adapted for the detection of CFTR (Cystic Fibrosis related) genes in human DNA. The peptide-nucleic acid probe conjugates 2 of the figures will be hereafter called the "activator oligonucleotides" whilst the ligand-associated nucleic acid probes 3 of the figures will be hereafter referred to as the "blocker oligonucleotides". The enzyme system used was beta-galactosidase whereby the alpha peptide complements the M15 mutant of this enzyme (Ullmann et al, J. Mol. Biol., 24 (1967) p339) to restore beta-galactosidase activity.

### I. Preparation of Activator Oligonucleotide

The activator oligonucleotide probes included the following nucleotide sequences:

All oligonucleotides were synthesised on an Applied Biosystems 380A synthesiser with nucleoside phosphoramidites. CFTR (A) and CFA508 (A) were each synthesised with a primary 3'-aliphatic amine by synthesis on a 3'-amino-ON CPG support supplied by Cruachem (UK) and using manufacturer's instructions. All oligonucleotides were purified by reverse-phase HPLC on a Unimetrics 5u RP-8 column using a linear-gradient of 7-35% acetonitrile in 100mM sodium acetate. 100µg each of CFTR (A) and CFA508 (A) were 5'-end labelled with trace amounts of 32P using 0.1µCi 32PATP (Amersham, 3000Ci/mmol) and 100 units of T4 polynucleotide kinase (Boehringer) in 200µl using conditions recommended with the enzyme. The labelled oligonucleotides were then heated to 80°C for 10 minutes, extracted twice with buffer equilibrated phenol (Gibco BRL) and chloroform (1:1), extracted once with chloroform, made 0.3M in sodium acetate pH5.2 and precipitated with 2 volumes of ice-cold ethanol. The pellets were washed once in 70% ethanol and lyophilised.

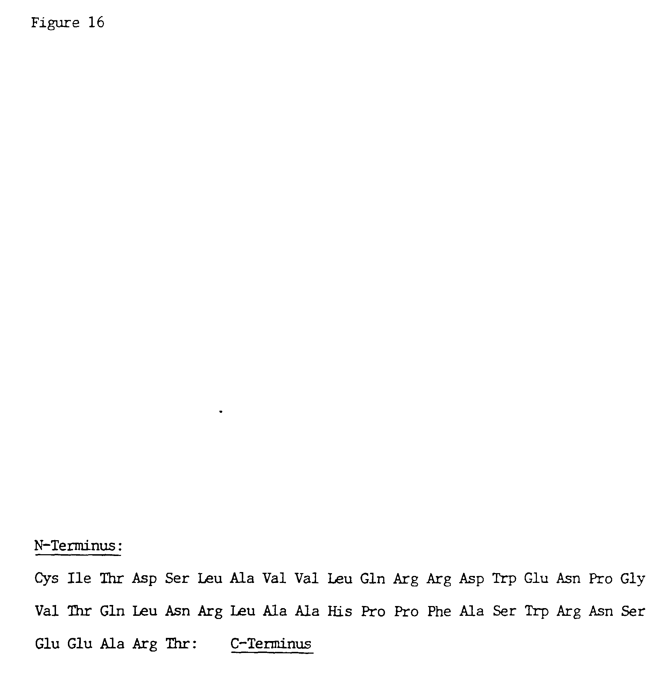

Two alternative forms of beta-galactosidase peptide were used, one comprising a 43 residue fragment with an N-terminal cysteine, herein called the cysteine/peptide, with an amino acid sequence detailed in figure 16 and the other comprising a plasmid derived alpha-peptide with an N-terminal methionine, herein called the methionine/peptide, as isolated from bacterial cells as detailed below. The cysteine/peptide was synthesised on an Applied Biosystems 431A peptide synthesiser using manufacturer's reagents and protocols and the peptide was purified using an Applied Biosystems ABS151A Separation System. The peptide was then dialysed into 1mM ammonium bicarbonate/1mM mercaptoethanol and lyophilised. The methionine/peptide was isolated from E.coli TG1 cells (obtained from Dr George Salmond, Dept Biological Sciences, University of Warwick, Coventry, UK) harbouring the plasmid pUC8 (obtained from CP Laboratories, Southampton, UK). Cells were grown in M9 medium (Molecular Cloning, supra) containing 100µg/ml ampicillin (sodium salt, Sigma, Poole, UK) and 0.02% w/v glucose. At A600nm = 0.4, IPTG (isopropyl thiogalactopyranoside, Sigma) was added to 1mM final concentration, cells were grown to A600 = 0.9, cells were pelleted and resuspended in 10ml of buffer A (0.05M Tris.HCl pH7.5, 0.1M NaCl, 0.01M MgCl2). The suspension was sonicated on ice using a Model XL20D Ultrasonicator (Heat Systems Inc, Farmingdale, New York, USA) at 6u peak to peak for five 20 second pulses at 60 second intervals. Cell debris was removed by centrifugation at 48,000g for 20 minutes at 4°C. The clear supernatent material was fractionated by adjusting to 50% saturation with solid ammonium sulphate and centrifuging at 18,000g for 10 minutes. The resulting ammonium sulphate pellet was resuspended in 2.5ml of buffer A and the solution was applied to a buffer A equilibrated PD10 column (Pharmacia, Milton Keynes, UK). Protein containing elution fractions were pooled and loaded onto a 2ml APTG-agarose column (p-aminophenyl beta-D-thiogalactopyranoside agarose, Sigma) and eluted with 0.1M sodium borate pH10.5. Protein containing fractions were pooled and tested for beta-galactosidase activity by the addition of 25µl of fraction to 1ml of 0.75mg/ml o-nitrophenyl-beta-D-galactopyranoside (ONPG, Sigma) in buffer A, incubation at 37°C for 30 minutes and the measurement of optical absorbance at 414nm. Beta-galactosidase containing fractions were treated with 8mol/litre urea and then run on a 10-15% SDS-polyacrylamide gradient gel. Gel slices below 14kD were excised and materials eluted into buffer A. Fractions were tested for the presence of methionine/peptide by mixing 25µl aliquots with 25µl (125 pmoles) of the M15 mutant of beta-galactosidase (prepared by the method of Langley et al., J. Biol. Chem., vol. 250 (1975) p2587-2592) in 1mM magnesium sulphate, 0.18mM manganese sulphate, 1mM ethylene diamine tetraacetic acid and 0.5M sodium phosphate buffer, pH7. The mixture was incubated for 30 minutes at 37°C and the optical absorbance at 414nm was measured.

For conjugation of oligonucleotide to cysteine/peptide, 100µg of each 3'-amino-oligonucleotide and 5mg of m-maleimidobenzoyl-N-hydroxysuccinimide ester (MBS, Pierce) were dissolved in 1ml of anhydrous tetrahydrofuran. To this solution was added 10mg of sodium carbonate and the reaction was allowed to proceed for 30 minutes. The mixture was then diluted 1:1 in 0.2M sodium phosphate buffer, pH7 and applied to a 20ml Sephadex® G25 column (Pharmacia) using 0.1M sodium phoshate, pH7 as the running buffer. Peak 32P-labelled fractions were pooled, made 0.3M in sodium acetate, pH5.2 and precipitated with 2 volumes of ice-cold ethanol. The pellets were washed once in 70% ethanol and lypophilised. To the maleimido-oligonucleotide was added 10µg of cysteine/peptide in 200µl 0.1M sodium phosphate, pH7. The mixture was stirred for 1 hour at room temperature and the oligonucleotide-peptide conjugate was then purified by anion exchange HPLC using a DuPont Zorbax GF250 column and an elution buffer of 0.2M potassium phosphate, pH7.

For conjugation of oligonucleotide to methionine/peptide, 100µg of each 3'-amino-oligonucleotide was dissolved in 25µl 0.1M sodium borate pH9.3 and 10mg of diisothiocyanate (DITC, Pierce, dissolved in 0.5ml dimethylformamide) was added. The mixture was shaken gently for 2 hours in the dark and further mixed with 6ml 50% (v/v) butan-1-ol. The mixture was shaken vigourously and centrifuged at 3000g for 5 minutes. The activated oligonucleotide solution was then further extracted with butan-1-ol to reduce the volume to <50µl and then dessicated. Methionine/alpha peptide was concentrated into 0.1M sodium borate pH9.3 using a Minicon CS15 concentrator (Amicon). 100µg (50µl) was mixed with the activated oligonucleotide preparation and incubated for 18 hours in the dark. Peptide/oligonucleotide conjugate was purified by electrophoresis on a 12.5% Swank Munkres SDS-polyacrylamide gel (Anal. Biochem., 99 (1979) p170) using trace 5'-32P labelling of oligonucleotide (Molecular Cloning, supra) in order to identify the conjugate band which was then excised, eluted into buffer A and dialysed in buffer A.

### II. Preparation of Blocker Oligonucleotides

The blocker oligonucleotides comprised the following oligonucleotide sequences complementary to the corresponding activator oligonucleotides (CFTR (B) and CFA508 (B)) and to a site on the CFTR gene adjacent to that complementary to the activator oligonucleotides (CFTR(C));

These oligonucleotides were synthesised with a 5'-bromodeoxyuridine nucleoside by incorporation of a 5-bromodeoxyuridine cyanoethyl phosphoramidite (Cruachem) into the synthesis.

### III.Preparation of Target DNA

DNA samples were obtained from a normal individual homozygous for the non-mutated CFTR (cystic fibrosis transregulator) gene and from an individual afflicted with cystic fibrosis by virtue of a homozygous deletion of codon 508 in the CFTR gene. DNA was derived from buccal cells obtained from these individuals by gentle scraping of the buccal lining with a sterile toothpick. Buccal cells were then suspended in 10µl sterile water, lysed with 20µl 0.1M potassium hydroxide and 0.1% Triton®-X-100 at 65°C for 20 minutes, and neutralised with 20µl of 0.1M HCl and 0.1% Triton®-X-100.

Regions of the CFTR gene were then amplified by the polymerase chain reaction (PCR) procedure as described in EP200362 (Cetus Corp). 5µl of the human cell lysate was denatured at 94°C for 3 minutes and mixed into 5µl of 20mM Tris.HCl pH8.3, 100mM potassium chloride, 3mM magnesium chloride, 20ng/ml bovine serum albumin (fraction V, Sigma), 400µM deoxyribonucleotide mixture (Pharmacia), 0.1% Triton®-X-100, 0.05 units Thermus aquaticus DNA polymerase (Perkin Elmer) and 0.5µM oligonucleotide amplification primers (5'-CAGTGGAAGAATGGCATTCTGTT-3' and 5'-GGCATGCTTTGATGACGCTTCTG-3'). Amplification was undertaken by 40 cycles in a thermal cycler (Techne, model PHC-2) comprising successive steps of 93°C for 1 minute, 55°C for 1.5 minutes and 72°C for 3 minutes. An additional 0.05 units of polymerase was added at the 20 cycle stage.

### IV.Hybridisation Analysis with Anti-Activator Antibody

Hybridisations analyses were undertaken as illustrated in figures 12 and 13. Hybridisation reactions comprised mixtures of DNA derived from normal individuals (confirmed homozygous wild-type CFTR gene), prepared as described in III. above, with the oligonucleotide combination CFTR(A) and CFTR(B). In addition, controls comprised mixtures without human DNA. For hybridisation, 10 pmoles of methionine peptide/oligonucleotide conjugate was mixed with dilutions of target human DNA (or PCR buffer blank) in 50µl of 5% formamide, 0.6M sodium chloride and 0.06M sodium citrate at pH7 and mixtures were incubated at 37°C for 30 minutes. 50µl of a 1:5 dilution in 0.15M NaCl and 0.015M sodium citrate pH7 of anti-single stranded DNA antibody (clone number 1619, Biogenesis, Bournemouth, UK) was added and the incubation continued for a further 30 minutes.

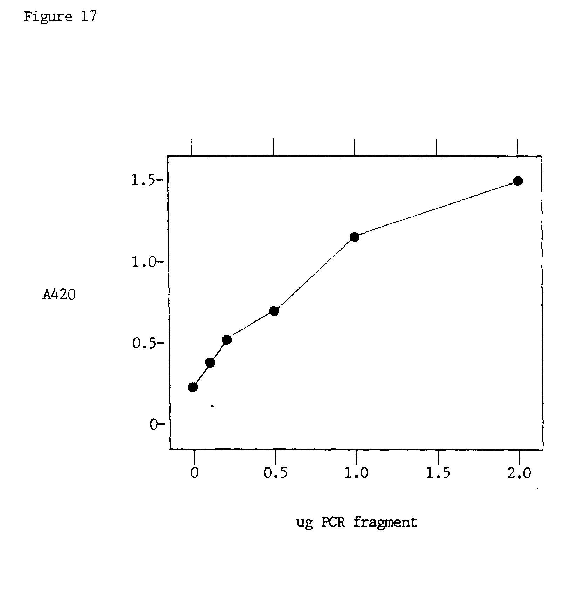

Beta-galactosidase activity was measured as follows. To the above mixture was added 900µl of a mixture comprising 1mg/ml ONPG, 125 pmoles of M15 preparation in 1mM magnesium sulphate, 0.18mM manganese sulphate, 1mM ethylene diamine tetraacetic acid and 0.5M sodium phosphate buffer, pH7. The mixture was incubated for 30 minutes at 37°C and the optical absorbance at 420nm was measured. The results are shown in figure 17 and show that the introduction of human DNA results in an increased beta-galactosidase activity than in the control.

### V. Hybridisation Analysis with Complementary Blocker Oligonucleotide

Hybridisation analyses were undertaken as illustrated in figures 2 and 3. Hybridisation reactions comprised mixtures of DNA, prepared as described in III. above, derived from normal or cystic fibrosis afflicted individuals either with the oligonucleotide combination CFTR(A) and CFTR(B) or the combination CFA508(A) and CFA508(B). In addition, controls comprised mixtures without human DNA. For hybridisation, 10 pmoles of cysteine peptide/oligonucleotide conjugate was mixed with 20 pmoles of blocker oligonucleotide and 2µl of human DNA (or PCR buffer blank) in 50µl of 5% formamide, 0.6M sodium chloride and 0.06M sodium citrate at pH7 and mixtures were incubated at 37°C for 30 minutes. 20µl of an anti-bromodeoxyuridine antibody (Amersham) diluted 1:2 in 10µl 0.6M sodium chloride was added and the incubation continued for a further 30 minutes.

Beta-galactosidase activity was measured as follows. To the above mixture was added 440µl of a mixture comprising 1mg/ml ONPG, 125 pmoles of M15 preparation in 1mM magnesium sulphate, 0.18mM manganese sulphate, 1mM ethylene diamine tetraacetic acid and 0.5M sodium phosphate buffer, pH7. The mixture was incubated for 30 minutes at 37°C and the optical absorbance at 414nm was measured. The results are expressed as percentage beta-galactosidase (beta-gal) activity of that with the CFTR(A) activator in the absence of blocker oligonucleotide and were as follows;

| Activator/Blocker | Human DNA | % Beta-Gal Activity |
|---|---|---|
| | | |
| CFTR(A) /none | none | 100 (A414=1.16) |
| CFA508(A)/none | none | 89 |
| | | |
| CFTR(A) /CFTR(B) | none | 8 |
| CFA508(A)/CFA508(B) | none | 4 |
| | | |
| CFTR(A) /CFTR(B) | normal | 66 |
| CFTR(A) /CFTR(B) | cystic fibrosis | 28 |
| | | |
| CFA508(A)/CFA508(B) | normal | 33 |
| CFA508(A)/CFA508(B) | cystic fibrosis | 74 |

These results demonstrate discrimination of normal and cystic fibrosis DNA through a simple homogeneous beta-galactosidase activation assay.

### VI.Hybridisation Analysis with Adjacent Blocker Oligonucleotide

Hybridisation analyses were undertaken as illustrated in figures 14 and 15. Hybridisation reactions comprised mixtures of normal human DNA (confirmed homozygous CFTR gene) prepared as described in III. above with the oligonucleotide combination CFTR(A) and CFTR(C). In addition, controls comprised mixtures without human DNA. For hybridisation, 10 pmoles of cysteine peptide/oligonucleotide conjugate CFTR(A) was mixed with 10 pmoles of blocker oligonucleotide CFTR(C) and dilutions of human DNA (or PCR buffer blank) in 50µl of 5% formamide, 0.6M sodium chloride and 0.06M sodium citrate at pH7 and mixtures were incubated at 37°C for 30 minutes. 20µl of an anti-bromodeoxyuridine antibody (Amersham) diluted 1:2 in 10µl 0.6M sodium chloride was added and the incubation continued for a further 30 minutes.

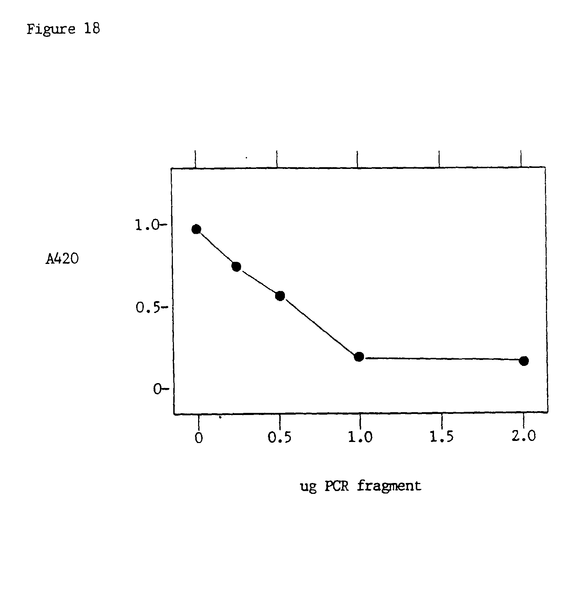

Beta-galactosidase activity was measured as described in V. above and the results, as shown in figure 18, show that the introduction of human DNA results in a dose dependant decrease in beta-galactosidase activity.

### Example 2 - HOMOGENEOUS ENZYME ACTIVATION ASSAY FOR p53 GENES

In this example, the enzyme activation assays as referred to in figures 1 to 3 and as described in example 1 was applied for the detection of the human p53 anti-oncogene.

### I. Preparation of Activator Oligonucleotide

The activator oligonucleotide probes included the following nucleotide sequences:

Oligonucleotides were synthesised and conjugated to cysteine/peptide as in example 1 above.

### II. Preparation of Blocker Oligonucleotides

The blocker oligonucleotides comprised the following oligonucleotide sequences complementary to the corresponding activator oligonucleotides;

These oligonucleotides were synthesised with a 5'-bromodeoxyuridine nucleotide as in example 1.

### III.Preparation of Target DNA

DNA samples were obtained from the human lymphoma cell line RAJI (ATCC7936) which is homozygous for a tract of p53 sequence complementary to p53(A) and (B) as above. 10⁴ cells were suspended in 100µl sterile water, lysed with 200µl 0.1M potassium hydroxide and 0.1% Triton®-X-100 at 65°C for 20 minutes, and neutralised with 200µl of 0.1M HCl and 0.1% Triton®-X-100.

Regions of the p53 gene were then amplified by the polymerase chain reaction (PCR) procedure as described in example 1. 5µl of the human cell lysate was denatured at 94°C for 3 minutes and mixed into 5µl of 20mM Tris.HCl pH8.3, 100mM potassium chloride, 3mM magnesium chloride, 20ng/ml bovine serum albumin (fraction V, Sigma), 400µM deoxyribonucleotide mixture (Pharmacia), 0.1% Triton®-X-100, 0.05 units Thermus aquaticus DNA polymerase (Perkin Elmer) and 0.5µM oligonucleotide amplification primers (5'-ATGCGAATTCCCCTGCCCTCAACAAGAT-3' and 5'-TATAGGAATTCGTGGTGAGGCTCCCCTT-3'). Amplification was undertaken by 35 cycles in a thermal cycler (Techne, model PHC-2) comprising successive steps of 94°C for 1 minute, 55°C for 2 minutes and 72°C for 3 minutes.

### IV.Hybridisation Analysis with Complementary Blocker Oligonucleotide

Hybridisation analyses were undertaken as illustrated in figures 2 and 3. Hybridisation reactions comprised mixtures of DNA, prepared as described in III. above, either with the oligonucleotide combination p53(A) and p53(B), the combination p53mut(A) and p53mut(B) or activator oligonucleotides alone. In addition, controls comprised mixtures without human DNA. For hybridisation, 10 pmoles of cysteine peptide/oligonucleotide conjugate was mixed with 20 pmoles of blocker oligcnucleotide and 2µl of human DNA (or PCR buffer blank). Hybridisation conditions, addition of anti-bromodeoxyuridine antibody and measurement of beta-galactosidase activity was as in example 1 above. The results were as follows;

| Activator/Blocker | Human DNA | A414nm (Beta-Gal Activity) |
|---|---|---|
| p53(A) /p53(B) | 2µl | 0.96 |
| p53(A) /p53(B) | none | 0.16 |
| p53(A) | 2µl | 1.23 |
| p53(A) | none | 1.33 |
| | | |
| p53mut(A)/p53mut(B) | 2µl | 0.38 |
| p53mut(A)/p53mut(B) | none | 0.18 |
| p53mut(A) | 2µl | 0.94 |
| p53mut(A) | none | 1.17 |

These results demonstrate the detection of the normal p53 anti-oncogene (combination of p53(A) and (B) oligonucleotides) through a simple homogeneous beta-galactosidase activation assay.

It will be apparent from the foregoing to those skilled in the art that a large number of different assay formats are envisaged within the scope of the present invention.

These include assays in which the sequence of interest competes with one probe for binding to the other probe and assays in which the sequence of interest is capable of binding to both probes.

The assay method of the present invention requires a detectable change in the behaviour of an enzyme. Such changes include the generation of enzyme activity.

Thus probes may be joined to enzyme fragments, enzyme activators or enzyme cofactors.

The sequence of interest may be a synthetic nucleic acid, a nucleic acid obtained from a prokaryotic or eukaryotic cell or derived by polymerase chain reaction. Typically, its size will be in the range from 10-10,000 nucleotides long.

Similarly, the probes may be synthetic oligonucleotides or derived from substantially natural sources. They may comprise synthetic base analogues, such as bromodeoxyuridine, which act as the ligand for a binding partner (such as anti-bromodeoxyuridine antibodies).

## Claims

1. A homogeneous assay method for determining the presence of a nucleic acid sequence of interest in a sample, the method comprising the steps of:
(a) combining in solution the sample, a first probe comprising single stranded nucleic acid substantially complementary to at least part of the sequence of interest and associated with a substance capable of activating an enzyme, and a second probe having specific binding activity for the first probe when the first probe is not bound to the sequence of interest;
(b) adding an enzyme capable of being activated by the substance associated with the first probe, such that in the absence of the sequence of interest the second probe binds to the first probe, wherein such binding inhibits activation of the enzyme by the substance associated with the first probe; and
(c) monitoring said enzyme activity.

2. A homogeneous assay method for determining the presence of a nucleic acid sequence of interest in a sample, the method comprising the steps of:
(a) combining in solution the sample, a first probe comprising single stranded nucleic acid substantially complementary to at least part of the sequence of interest and associated with a substance capable of activating an enzyme, and a second probe comprising a ligand moiety and single stranded nucleic acid complementary to the first probe, such that the presence of the sequence of interest in the sample tends to inhibit binding of the second probe to the first probe;
(b) adding an enzyme which is activatable by the substance associated with the first probe, and a binding partner capable of binding to the ligand moiety, such that when the first and second probes are bound to each other, binding of the binding partner to the ligand moiety inhibits activation of the enzyme by the substance associated with the first probe; and
(c) monitoring said enzyme activity.

3. A homogeneous assay method for determining the presence of a nucleic acid sequence of interest in a sample, the method comprising the steps of:
(a) combining in solution the sample, a first probe comprising single stranded nucleic acid substantially homologous with at least part of the sequence of interest and associated with a substance capable of activating an enzyme, and a second probe comprising a ligand moiety and single stranded nucleic acid complementary to both at least part of the sequence of interest and to the nucleic acid present in the first probe, such that the presence of the sequence of interest in the sample tends to inhibit binding of the second probe to the first probe;
(b) adding an enzyme which is activatable by the substance associated with the first probe, and a binding partner capable of binding to the ligand moiety, such that when the first and second probes are bound to each other, binding of the binding partner to the ligand moiety inhibits activation of the enzyme by the substance associated with the first probe; and
(c) monitoring said enzyme activity.

4. A homogeneous assay method for determining the presence of a nucleic acid sequence of interest in a sample, the method comprising the steps of:
(a) combining in solution the sample, a first probe comprising single stranded nucleic acid substantially complementary to at least part of the sequence of interest and associated with a substance capable of activating an enzyme, and a second probe comprising a ligand moiety and single stranded nucleic acid complementary to at least part of the sequence of interest, such that the presence of the sequence of interest in the sample tends to cause binding of the first and second probes in close proximity to each other on the sequence of interest;
(b) adding an enzyme which is activatable by the substance associated with the first probe, and a binding partner capable of binding to the ligand moiety, such that when the first and second probes are bound in close proximity to each other, binding of the binding partner to the ligand moiety inhibits activation of the enzyme by the substance associated with the first probe; and
(c) monitoring said enzyme activity.

5. A method according to claim 1, wherein the second probe is an antibody directed against the first probe.

6. A method according to claim 5, wherein the first probe comprises a synthetic base analogue and the second probe is an antibody directed against said synthetic base analogue.

7. A method according to claim 6, wherein said synthetic base analogue comprises bromodeoxyuridine.

8. A method according to any one of claims 2, 3 or 4, wherein the ligand moiety comprises a synthetic base analogue, and the binding partner is an antibody directed against said base analogue.

9. A method according to claim 8, wherein the ligand moiety comprises bromodeoxyuridine.

10. A method according to any one of claims 1 to 9, wherein the first probe is associated with an enzyme fragment, enzyme activator or enzyme co-factor.

11. A method according to claim 10, wherein the first probe is associated with a fragment of beta-galactosidase or RNase S.

12. A method according to claim 11, wherein the first probe is associated with the M15 mutant fragment of β-galatctosidase.

13. A method according to claim 11, wherein the first probe is associated with a fragment of β-galactosidase comprising the amino acid sequence shown in Figure 16 (Seq. ID No. 14), or functional equivalents thereof.

14. A method according to claim 10, wherein the first probe is associated with glucose-6-phosphate dehydrogenase.

15. A method according to any one of the preceding claims, wherein the sequence of interest comprises any nucleic acid greater than 10 nucleotides long.

16. A method according to claim 15, wherein the sequence of interest comprises any nucleic acid less than 10,000 nucleotides long.

17. A method according to any one of the preceding claims wherein the enzyme behaviour is monitored quantitatively.

18. A kit for performing the method of any one of claims 2, 3 or 4, comprising a first probe, a second probe, an enzyme substrate and a binding partner as defined in said claims.

## Patentansprüche

1. Homogenes Nachweisverfahren zur Bestimmung des Vorliegens einer interessierenden Nukleinsäuresequenz in einer Probe, wobei das Verfahren die Schritte umfaßt:
(a) Kombinieren in Lösung der Probe, einer ersten Sonde, die eine einzelsträngige Nukleinsäure umfaßt, die im wesentlichen zumindest zu einem Teil der interessierenden Sequenz komplementär ist und mit einer zum Aktivieren eines Enzyms fähigen Substanz assoziiert ist, und einer zweiten Sonde, die eine spezifische Bindungsaktivität für die erste Sonde aufweist, wenn die erste Sonde nicht an die interessierende Sequenz gebunden ist;
(b) Zugeben eines Enzyms, das in der Lage ist, von der mit der ersten Sonde assoziierten Substanz aktiviert zu werden, so daß in Abwesenheit der interessierenden Sequenz die zweite Sonde an die erste Sonde bindet, wobei diese Bindung die Aktivierung des Enzyms durch die Substanz, die mit der ersten Sonde assoziiert ist, inhibiert und
(c) Überwachen der Enzymaktivität.

2. Homogenes Nachweisverfahren zur Bestimmung des Vorliegens einer interessierenden Nukleinsäuresequenz in einer Probe, wobei das Verfahren die Schritte umfaßt:
(a) kombinieren in Lösung der Probe, einer ersten Sonde, die eine einzelsträngige Nukleinsäure umfaßt, die zumindest zu einem Teil der interessierenden Sequenz komplementär ist und mit einer zum Aktivieren eines Enzyms fähigen Substanz assoziiert ist, und einer zweiten Sonde, die einen Ligandenteil und eine zur ersten Sonde komplementäre einzelsträngige Nukleinsäuresequenz umfaßt, so daß das Vorliegen der interessierenden Sequenz in der Probe zu einer Inhibierung der Bindung der zweiten Sonde an die erste Sonde führt;
(b) Zugeben eines Enzyms, das durch die mit der ersten Sonde assoziierte Substanz aktivierbar ist, und eines zur Bindung an den Ligandenteil fähigen Bindungspartners, so daß, falls die erste und die zweite Sonde aneinander gebunden sind, die Bindung des Bindungspartners an den Ligandenteil die Aktivierung des Enzyms durch die mit der ersten Sonde assoziierte Substanz inhibiert und
(c) Überwachen der Enzymaktivität.

3. Homogenes Nachweisverfahren zur Bestimmung des Vorliegens einer interessierenden Nukleinsäuresequenz in einer Probe, wobei das Verfahren die Schritte umfaßt:
(a) Kombinieren in Lösung der Probe, einer ersten Sonde, die eine einzelsträngige Nukleinsäure umfaßt, die im wesentlichen zumindest zu einem Teil der interessierenden Sequenz homolog ist und mit einer zur Aktivierung eines Enzyms fähigen Substanz assoziiert ist, und einer zweiten Sonde, die einen Ligandenteil und eine einzelsträngige Nukleinsäure umfaßt, die sowohl zumindest zu einem Teil der interessierenden Sequenz, als auch zu der in der ersten Sonde vorliegenden Nukleinsäure komplementär ist, so daß das vorliegen der interessierenden Sequenz in der Probe zur Inhibierung der Bindung der zweiten Sonde an die erste Sonde führt;
(b) Zugeben eines Enzyms, das durch die mit der ersten Sonde assoziierte Substanz aktivierbar ist, und eines zur Bindung an den Ligandenteil fähigen Bindungspartners, so daß, falls die erste und zweite Sonde aneinander gebunden sind, die Bindung des Bindungspartners an den Ligandenteil die Aktivierung des Enzyms durch die mit der ersten Sonde assoziierte Substanz inhibiert und
(c) Überwachen der Enzymaktivität.

4. Homogenes Nachweisverfahren zur Bestimmung des Vorliegens einer interessierenden Nukleinsäuresequenz in einer Probe, wobei das Verfahren die Schritte umfaßt:
(a) Kombinieren in Lösung der Probe, einer ersten Sonde, die eine einzelsträngige Nukleinsäure umfaßt, die im wesentlichen zumindest zu einem Teil der interessierenden Sequenz komplementär ist und mit einer zur Aktivierung eines Enzyms fähigen Substanz assoziiert ist, und einer zweiten Sonde, die einen Ligandenteil und eine einzelsträngige Nukleinsäure umfaßt, die zumindest zu einem Teil der interessierenden Sequenz komplementär ist, so daß das Vorliegen der interessierenden Sequenz in der Probe zur Bindung der ersten und zweiten Sonde in großer Nähe zueinander an der interessierenden Sequenz führt;
(b) Zugeben eines Enzyms, das durch die mit der ersten Sonde assoziierte Substanz aktivierbar ist, und eines zur Bindung an den Ligandenteil fähigen Bindungspartners, so daß, falls die erste und die zweite Sonde in großer Nähe zueinander gebunden sind, die Bindung des Bindungspartners an den Ligandenteil die Aktivierung des Enzyms durch die mit der ersten Sonde assoziierte Substanz inhibiert und
(c) Überwachen der Enzymaktivität.

5. Verfahren nach Anspruch 1, wobei die zweite Sonde ein gegen die erste Sonde gerichteter Antikörper ist.

6. Verfahren gemäß Anspruch 5, wobei die erste Sonde ein synthetisches Basenanalogon umfaßt und die zweite Sonde ein gegen das synthetische Basenanalogon gerichteter Antikörper ist.

7. Verfahren gemäß Anspruch 6, wobei das synthetische Basenanalogon Bromdeoxyuridin umfaßt.

8. Verfahren nach mindestens einem der Ansprüche 2, 3 oder 4, wobei der Ligandenteil ein synthetisches Basenanalogon umfaßt und der Bindungspartner ein gegen das Basenanalogon gerichteter Antikörper ist.

9. Verfahren gemäß Anspruch 8, wobei der Ligandenteil Bromdeoxyuridin umfaßt.

10. Verfahren gemäß mindestens einem der Ansprüche 1 bis 9, wobei die erste Sonde mit einem Enzymfragment, Enzymaktivator oder Enzymcofaktor assoziiert ist.

11. Verfahren gemäß Anspruch 10, wobei die erste Sonde mit einem Fragment der β-Galactosidase oder der RNase S assoziiert ist.

12. Verfahren gemäß Anspruch 11, wobei die erste Sonde mit dem M15-Mutantenfragment der β-Galactosidase assoziiert ist.

13. Verfahren gemäß Anspruch 11, wobei die erste Sonde mit einem Fragment der β-Galactosidase assoziiert ist, die die in Figur 16 (Sequenz ID Nr. 14) gezeigte Aminosäuresequenz umfaßt, oder mit funktionellen Äquivalenten dieser.

14. Verfahren gemäß Anspruch 10, wobei die erste Sonde mit Glucose-6-phosphatdehydrogenase assoziiert ist.

15. Verfahren gemäß mindestens einem der vorhergehenden Ansprüche, wobei die interessierende Sequenz eine beliebige Nukleinsäure mit mehr als 10 Nukleotiden Länge umfaßt.

16. Verfahren gemäß Anspruch 15, wobei die interessierende Sequenz eine beliebige Nukleinsäure mit weniger als 10.000 Nukleotiden Länge umfaßt.

17. Verfahren gemäß mindestens einem der vorhergehenden Ansprüche, wobei das Verhalten des Enzyms quantitativ überwacht wird.

18. Kit zum Ausführen des Verfahrens nach mindestens einem der Ansprüche 2, 3 oder 4, umfassend eine erste Sonde, eine zweite Sonde, ein Enzymsubstrat und einen Bindungspartner, wie in diesen Ansprüchen definiert.

## Revendications

1. Méthode de test homogène pour déterminer la présence d'une séquence d'acide nucléique intéressante dans un échantillon, la méthode comprenant les étapes consistant :
(a) à combiner en solution l'échantillon, une première sonde comprenant un acide nucléique monocaténaire sensiblement complémentaire d'au moins une partie de la séquence intéressante et associé avec une substance capable d'activer une enzyme, et une seconde sonde ayant une activité de liaison spécifique de la première sonde lorsque la première sonde n'est pas liée à la séquence intéressante;
(b) à ajouter une enzyme pouvant être activée par la substance associée avec la première sonde, de telle sorte qu'en l'absence de la séquence intéressante, la seconde sonde se lie à la première sonde, cette liaison inhibant l'activation de l'enzyme par la substance associée à la première sonde ; et
(c) à suivre ladite activité de l'enzyme.

2. Méthode de test homogène pour déterminer la présence d'une séquence d'acide nucléique intéressante dans un échantillon, la méthode comprenant les étapes consistant :
(a) à combiner en solution l'échantillon, une première sonde comprenant un acide nucléique monocaténaire sensiblement complémentaire d'au moins une partie de la séquence intéressante et associé avec une substance capable d'activer une enzyme, et une seconde sonde comprenant une partie ligand et un acide nucléique monocaténaire complémentaire de la première sonde, de telle sorte que la présence de la séquence intéressante dans l'échantillon ait tendance à inhiber la liaison de la seconde sonde à la première sonde ;
(b) à ajouter une enzyme pouvant être activée par la substance associée avec la première sonde et un partenaire de liaison capable de se lier à la partie ligand, de telle sorte que lorsque la première et la seconde sondes sont liées l'une à l'autre, la liaison du partenaire de liaison à la partie ligand inhibe l'activation de l'enzyme par la substance associée à la première sonde ; et
(c) à suivre ladite activité de l'enzyme.

3. Méthode de test homogène pour déterminer la présence d'une séquence d'acide nucléique intéressante dans un échantillon, la méthode comprenant les étapes consistant :
(a) à combiner en solution l'échantillon, une première sonde comprenant un acide nucléique monocaténaire sensiblement homologue d'au moins une partie de la séquence intéressante et associé avec une substance capable d'activer une enzyme, et une seconde sonde comprenant une partie ligand et un acide nucléique monocaténaire complémentaire à la fois d'au moins une partie de la séquence intéressante et de l'acide nucléique présent dans la première sonde, de telle sorte que la présence de la séquence intéressante dans l'échantillon ait tendance à inhiber la liaison de la seconde sonde à la première sonde ;
(b) à ajouter une enzyme pouvant être activée par la substance associée avec la première sonde et un partenaire de liaison capable de se lier à la partie ligand, de telle sorte que lorsque la première et la seconde sondes sont liées l'une à l'autre, la liaison du partenaire de liaison à la partie ligand inhibe l'activation de l'enzyme par la substance associée à la première sonde ; et
(c) à suivre ladite activité de l'enzyme.

4. Méthode de test homogène pour déterminer la présence d'une séquence d'acide nucléique intéressante dans un échantillon, la méthode comprenant les étapes consistant :
(a) à combiner en solution l'échantillon, une première sonde comprenant un acide nucléique monocaténaire sensiblement complémentaire d'au moins une partie de la séquence intéressante et associé avec une substance capable d'activer une enzyme, et une seconde sonde comprenant une partie ligand et un acide nucléique monocaténaire complémentaire d'au moins une partie de la séquence intéressante, de telle sorte que la présence de la séquence intéressante dans l'échantillon ait tendance à provoquer la liaison de la première et de la seconde sondes en proximité étroite l'une de l'autre sur la séquence intéressante ;
(b) à ajouter une enzyme pouvant être activée par la substance associée avec la première sonde et un partenaire de liaison capable de se lier à la partie ligand, de telle sorte que lorsque la première et la seconde sondes sont liées en étroite proximité l'une de l'autre, la liaison du partenaire de liaison à la partie ligand inhibe l'activation de l'enzyme par la substance associée à la première sonde ; et
(c) à suivre ladite activité de l'enzyme.

5. Méthode selon la revendication 1, dans laquelle la seconde sonde est un anticorps dirigé contre la première sonde.

6. Méthode selon la revendication 5, dans laquelle la première sonde comprend un analogue de base synthétique et la seconde sonde est un anticorps dirigé contre ledit analogue de base synthétique.

7. Méthode selon la revendication 6, dans laquelle ledit analogue de base synthétique comprend de la bromodésoxyuridine.

8. Méthode selon l'une quelconque des revendications 2, 3 ou 4, dans laquelle la partie ligand comprend un analogue de base synthétique, et le partenaire de liaison est un anticorps dirigé contre ledit analogue de base.

9. Méthode selon la revendication 8, dans laquelle la partie ligand comprend de la bromodésoxyuridine.

10. Méthode selon l'une quelconque des revendications 1 à 9, dans laquelle la première sonde est associée avec un fragment d'enzyme, un activateur d'enzyme ou un co-facteur d'enzyme.

11. Méthode selon la revendication 10, dans laquelle la première sonde est associée avec un fragment de bêta-galactosidase ou de RNase S.

12. Méthode selon la revendication 11, dans laquelle la première sonde est associée avec le fragment de bêta-galactosidase du mutant M15.

13. Méthode selon la revendications 11, dans laquelle la première sonde est associée avec un fragment de β-galactosidase comprenant la séquence d'acides aminés présentée à la figure 16 (Seq. ID No. 14), ou l'un de ses équivalents fonctionnels.

14. Méthode selon la revendication 10, dans laquelle la première sonde est associée avec la glucose-6-phosphate déshydrogénase.

15. Méthode selon l'une des revendications précédentes, dans laquelle la séquence intéressante comprend un acide nucléique quelconque de longueur supérieure à 10 nucléotides.

16. Méthode selon la revendications 15, dans laquelle la séquence intéressante comprend un acide nucléique quelconque de longueur inférieure à 10 000 nucléotides.

17. Méthode selon l'une quelconque des revendications précédentes, dans laquelle le comportement de l'enzyme est suivi quantitativement.

18. Kit pour mettre en oeuvre la méthode de l'une quelconque des revendications 2, 3 ou 4, comprenant une première sonde, une seconde sonde, un substrat d'enzyme et un partenaire de liaison, tels que définis dans lesdites revendications.


### SEQUENCE LISTING

(1) GENERAL INFORMATION:
(i) APPLICANT:
   (A) NAME: Cytocell Limited
   (B) STREET: Home Farm, Hill Road
   (C) CITY: Lewknor
   (D) STATE: Oxfordshire
   (E) COUNTRY: United Kingdom
   (F) POSTAL CODE (ZIP): 0X9 5TS
   (G) TELEPHONE: (0844) 353731
   (H) TELEFAX: (0844) 3522448
(ii) TITLE OF INVENTION: Nucleic Acids Assay
(iii) NUMBER OF SEQUENCES: 14
(iv) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25 (EPO)
(v) CURRENT APPLICATION DATA:
   APPLICATION NUMBER: EP 92914959.8
(2) INFORMATION FOR SEQ ID NO: 1:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 1:
   AATATCATCT TTGGTGTTTC 20
(2) INFORMATION FOR SEQ ID NO: 2:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 2:
   GAAAATATCA TCGGTGTTTC 20
(2) INFORMATION FOR SEQ ID NO: 3:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 3:
   GAAACACCAA AGATGATATT 20
(2) INFORMATION FOR SEQ ID NO: 4:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 4:
   GAAACACCGA TGATATTTTC 20
(2) INFORMATION FOR SEQ ID NO: 5:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 5:
   CTATGATGAA TATAGATACA 20
(2) INFORMATION FOR SEQ ID NO: 6:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 6:
   CAGTGGAAGA ATGGCATTCT GTT 23
(2) INFORMATION FOR SEQ ID NO: 7:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 7:
   GGCATGCTTT GATGACGCTT CTG 23
(2) INFORMATION FOR SEQ ID NO: 8:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 8:
   GGCATGAACC GGAGGCCC 18
(2) INFORMATION FOR SEQ ID NO: 9:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 9:
   GGCATGAACC AGAGGCCC 18
(2) INFORMATION FOR SEQ ID NO: 10:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 10:
   GGGCCTCCGG TTCATGCC 18
(2) INFORMATION FOR SEQ ID NO: 11:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 11:
   GGGCCTCTGG TTCATGCC 18
(2) INFORMATION FOR SEQ ID NO: 12:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 12:
   ATGCGAATTC CCCTGCCCTC AACAAGAT 28
(2) INFORMATION FOR SEQ ID NO: 13:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 13:
   TATAGGAATT CGTGGTGAGG CTCCCCTT 28
(2) INFORMATION FOR SEQ ID NO: 14:
   (i) SEQUENCE CHARACTERISTICS:
      (A) LENGTH: 43 amino acids
      (B) TYPE: amino acid
      (C) STRANDEDNESS: single
      (D) TOPOLOGY: unknown
   (ii) MOLECULE TYPE: peptide
   (iii) HYPOTHETICAL: NO
   (v) FRAGMENT TYPE: internal
   (xi) SEQUENCE DESCRIPTION: SEQ ID NO: 14:
   Cys Ile Thr Asp Ser Leu Ala Val Val Leu Gln Arg Arg Asp Trp
   1               5                   10                  15
   Glu Asn Pro Gly Val Thr Gln Leu Asn Arg Leu Ala Ala His Pro
                   20                  25                  30
   Pro Phe Ala Ser Trp Arg Asn Ser Glu Glu Ala Arg Thr
                   35                  40